# EUROPEAN PATENT APPLICATION

(11) **EP 3 438 252 A1**
(43) Date of publication of application: **06.02.2019**
(21) Application number: 18186010.7
(22) Date of filing: 27.07.2018
(51) Int. Cl.: C12N 9/12, C12P 19/04

(54) **RECOMBINANT MICROORGANISM OF GENUS KOMAGATAEIBACTER, METHOD OF PRODUCING CELLULOSE BY USING THE SAME, AND METHOD OF PRODUCING THE MICROORGANISM**

(30) Priority: 02.08.2017 KR 20170098071; 29.11.2017 KR 20170161834
(71) Applicant: Samsung Electronics Co., Ltd., Gyeonggi-do 16677 (KR)
(72) Inventor: CHUNG, Soonchun, 16678 Gyeonggi-do (KR); LEE, Sunghaeng, 16678 Gyeonggi-do (KR); PARK, Jinhwan, 16678 Gyeonggi-do (KR)
(74) Representative: Grünecker Patent- und Rechtsanwälte PartG mbB

(57) **Abstract**

Provided are a microorganism of the genus *Komagataeibacter* having enhanced cellulose productivity and yield, a method of producing cellulose by using the same, and a method of producing the microorganism.

## Description

### BACKGROUND

### 1. Field

The present disclosure relates to a microorganism of the genus *Komagataeibacter* comprising a genetic modification that increases expression of polyphosphate kinase (PPK) or an activity thereof, a method of producing cellulose by using the microorganism, and a method of producing the microorganism.

### 2. Description of the Related Art

Cellulose produced by culturing microorganisms, also known as "biocellulose" or "microbial cellulose," exists as a primary structure of β-1,4 glucan composed of glucose that forms a network structure of fibril bundles. Microbial cellulose is typically about 100 nm or less in width, and, unlike plant cellulose, is free of lignin or hemicellulose. Additionally, compared to plant cellulose, microbial cellulose has increased water absorption and retention capacity, higher tensile strength, higher elasticity, and higher heat resistance. Due to these characteristics, microbial cellulose has been developed for applications in a variety of fields, such as cosmetics, medical products, dietary fibers, audio speaker diaphragms, and functional films.

Therefore, there is a need to develop new microorganisms and methods to increase the production of microbial cellulose.

### SUMMARY

Provided is a microorganism of the genus *Komagataeibacter* comprising a genetic modification that increases expression or activity of polyphosphate kinase (PPK).

Also provided is a method of producing cellulose by using the microorganism, as well as a method of producing the microorganism.

### BRIEF DESCRIPTION OF THE DRAWINGS

These and/or other aspects will become apparent and more readily appreciated from the following description of the embodiments, taken in conjunction with the accompanying drawings in which:
FIG. 1 shows cellulose nanofiber (CNF) production of a *K. xylinus* strain into which a pfkA gene is introduced compared to control strains;
FIG. 2 shows CNF yield of a *K. xylinus* strain into which a pfkA gene is introduced compared to control strains;
FIG. 3 shows CNF production and yield of a *K. xylinus* strain into which a pfkA gene is introduced during fermentation in a medium free of carboxy methyl cellulose (CMC) compared to control strains;
FIG. 4 shows CNF production and yield of a *K.xylinus* strain into which a pfkA gene is introduced during fermentation in a medium including CMC compared to a control strain; and
FIG. 5 shows results of comparing CNF production of SK3 strains, in which the SK3 strains are introduced with the indicated ppk gene.

### DETAILED DESCRIPTION

The term "increase in expression", as used herein, may refer to an increase in transcription or translation of a gene encoding a protein or an enzyme.

The term "increase in activity" or "increased activity", or like terms, as used herein refers to a detectable increase in an activity level of a modified (e.g., genetically engineered) cell, protein, or enzyme compared to a cell, protein, or enzyme of the same type that does not have the given genetic modification (e.g., a parent cell or a native, original, or "wild-type" cell, protein, or enzyme). For example, an activity of a modified or engineered cell, protein, or enzyme may be increased by about 5% or more, about 10% or more, about 15% or more, about 20% or more, about 30% or more, about 50% or more, about 60% or more, about 70% or more, or about 100% or more relative to the activity of a non-engineered cell (e.g., parent cell), protein, or enzyme of the same type A cell having an increased activity of a protein or an enzyme may be identified by using any method known in the art.

A cell having increased activity of an enzyme or a polypeptide may be achieved by an increase in expression or specific activity of the enzyme or polypeptide. The increase in expression may be caused by introduction of a polynucleotide encoding the enzyme or the polypeptide into a cell, by otherwise increasing the copy number of the polynucleotide that encodes the enzyme or polypeptide, or by modification of a regulatory region of the polynucleotide encoding the enzyme or polypeptide so as to increase expression thereof. The introduction of a polynucleotide encoding the enzyme or polypeptide may be a transient introduction in which the gene is not integrated into a genome, or an introduction that results in integration of the gene into the genome. The introduction may be performed, for example, by introducing a vector comprising a polynucleotide encoding the enzyme or polypeptide into the cell.

A polynucleotide introduced into the cell may be operably linked to a regulatory sequence that allows expression of the enzyme or polypeptide, for example, a promoter, a polyadenylation site, or a combination thereof. When an exogenous polynucleotide is introduced, the polynucleotide may comprise a sequence that is endogenous or heterologous to the microorganism in which it is inserted. As used herein, an endogenous gene refers to a polynucleotide that is present in the intrinsic genetic material of the microorganism prior to a given genetic manipulation, for instance, a polynucleotide present in the genetic material of the wild-type or native microorganism. The term "heterologous" means "foreign" or "not native" to the species.

An increase in copy number of a polynucleotide refers to any increase in copy number. For example, an increase in copy number may be caused by introduction of an exogenous polynucleotide (whether endogenous or heterologous) or amplification of an endogenous polynucleotide. In one embodiment, an increase in copy number may be achieved by genetically engineering a cell so that the cell has a polynucleotide that does not exist in a non-engineered cell. The introduction of a polynucleotide may be a transient introduction in which the polynucleotide is not integrated into a genome, or an introduction that results in integration of the polynucleotide into the genome. The introduction may be performed, for example, by introducing a vector into the cell, the vector including a polynucleotide encoding a target polypeptide, and then, replicating the vector in the cell, or by integrating the polynucleotide into the genome.

The introduction of the gene may be performed via a known method, for example, transformation, transfection, or electroporation.

The term "vector" or "vehicle", as used herein, refers to a nucleic acid molecule that is able to deliver nucleic acids linked thereto into a cell. The vector may include, for example, a plasmid expression vector, a virus expression vector, such as a replication-defective retrovirus, adenovirus, or an adeno-associated virus.

The genetic modification used in the present disclosure may be performed by any molecular biological method known in the art.

The term "parent cell" refers to an original cell, for example, a non-genetically engineered cell of the same type as an engineered microorganism. With respect to a particular genetic modification, the "parent cell" may be a cell that lacks the particular genetic modification, but is identical in all other respects. Thus, the parent cell may be a cell that is used as a starting material to produce a genetically engineered microorganism having an increased activity of a given protein (e.g., a protein having a sequence identity of about 90% or higher with respect to phosphofructose kinase).

The term "gene" and "polynucleotide", as used herein are synonymous and refers to a nucleic acid fragment encoding a particular protein, and may optionally include a regulatory sequence of a 5'-non coding sequence and/or a 3'-non coding sequence.

The term "sequence identity" of a polynucleotide or a polypeptide, as used herein, refers to a degree of identity between bases or amino acid residues of sequences obtained after the sequences are aligned so as to best match in certain comparable regions. The sequence identity is a value that is measured by comparing two sequences in certain comparable regions via optimal alignment of the two sequences, in which portions of the sequences in the certain comparable regions may be added or deleted compared to reference sequences. A percentage of sequence identity may be calculated by, for example, comparing two optimally aligned sequences in the entire comparable regions, determining the number of locations in which the same amino acids or nucleic acids appear to obtain the number of matching locations, dividing the number of matching locations by the total number of locations in the comparable regions (that is, the size of a range), and multiplying a result of the division by 100 to obtain the percentage of the sequence identity. The percentage of the sequence identity may be determined using a known sequence comparison program, for example, BLASTN (NCBI), BLASTP (NCBI), CLC Main Workbench (CLC bio), MegAlign™ (DNASTAR Inc), etc.

Various levels of sequence identity may be used to identify various types of polypeptides or polynucleotides having the same or similar functions or activities. For example, the sequence identity may include a sequence identity of about 50% or more, about 55% or more, about 60% or more, about 65% or more, about 70% or more, about 75% or more, about 80% or more, about 85% or more, about 90% or more, about 95% or more, about 96% or more, about 97% or more, about 98% or more, about 99% or more, or 100%.

The term "genetic modification", as used herein, refers to an artificial alteration in a constitution or structure of a genetic material of a cell.

An aspect of the present disclosure provides a microorganism, such as a recombinant microorganism, including a genetic modification that increases phosphofructose kinase (PPK) activity. Polyphosphate kinase (PPK) is an enzyme that catalyzes a reversible reaction of converting NTP + (phosphate)n to NDP + (phosphate)n+1. Here, NTP and NDP represent nucleoside triphosphate and nucleoside diphosphate, respectively. NTP may be ATP, GTP, CTP, or UTP. NDP may be ADP, GDP, CDP, or UDP. In an embodiment, PPK catalyzes both the forward and reverse reactions, and may have higher catalytic activity for the reverse reaction than catalytic activity for the forward reaction than the reverse reaction. In another embodiment, PPK may have higher catalytic activity for conversion of NDP to NTP in a reaction using GDP, CDP, UDP, or a combination thereof as the substrate, compared to using ADP as the substrate. That is, PPK may convert NDP to NTP by using inorganic polyphosphate as a donor. NTP, particularly, UTP may be used in the synthesis of cellulose. PPK may have high catalytic activity for conversion of NDP to NTP in a reaction using UDP, GDP, or UDP and GDP as a substrate, compared to using ADP, in the reverse reaction. PPK may have the highest activity and selectivity for pyrimidine nucleoside diphosphate. This activity is also called polyphosphate-driven nucleoside diphosphate kinase (PNDK) activity. This activity may be measured by incubating a reaction mixture including 75 mM polyphosphate (as phosphate), 30 mM MgCl₂, 5 mM NDP, and 50 mM Tris-HCI (pH 7.8) at 30°C.

PPK may be heterologous or endogenous to the microorganism. The PPK may be an enzyme classified as EC 2.7.4.1. PPK may be from bacteria. For instance, PPK may be from the genus *Silicibacter* or the genus *Rhodobacterales.* More specficially, PPK may be from *Silicibacter pomeroyi, Silicibacter lacuscaerulensi,* or *Rhodobacterales bacterium.*

In an embodiment, the genetic modification that increases PPK activity, is the introduction of a polynucleotide encoding a PPK having an activity belonging to EC 2.7.4.1.

In another embodiment the genetic modification that increases PPK activity is the introduction of a polynucleotide encoding a polypeptide comprising an amino acid sequence having a sequence identity of about 90% or higher, about 95% or higher, or about 100% to the amino acid sequence of SEQ ID NO: 44, 46, or 48. For example, a polynucleotide comprising a nucleotide sequences having a sequence identity of about 90% or higher, about 95% or higher, or about 100% to the nucleotide sequence of SEQ ID NO: 45, 47, or 49.

The microorganism comprising the genetic modification that increases PPK activity may have enhanced cellulose productivity as compared to a microorganism of the same type without the genetic modification (e.g., as compared to a parent microorganism). The cellulose may also be called nanocellulose, cellulose nanofiber (CNF), microfibrillated cellulose (MFC), nanocrystalline cellulose (NCC), or bacterial nanocellulose. The cellulose may be cellulose free of lignin or hemicelluloses. A fiber width of the cellulose may be about 100 nm or less, about 90 nm or less, about 80 nm or less, about 70 nm or less, about 60 nm or less, about 50 nm or less, about 40 nm or less, about 30 nm or less, about 20 nm or less, or about 10 nm or less. The cellulose may have high absorbency, high strength, high elasticity, high heat resistance or a combination thereof.

The recombinant microorganism of the invention may further include a genetic modification that increases phosphofructose kinase (PFK) activity.

PFK is a protein that phosphorylates fructose-6-phosphate into fructose-1,6-bisphosphate in glycolysis. PFK may catalyze conversion of ATP and fructose-6-phosphate into fructose-1,6-bisphosphate and ADP. PFK is allosterically activated by ADP and diphosphonucleoside, and allosterically inhibited by phosphoenolpyruvate. The PFK may be heterologous or endogenous to the modified microorganism.

PFK may be PFK1 (also called "PFKA"). PFK1 may belong to the enzyme classified as EC 2.7.1.11. PFK may be derived from bacteria. PFK may be derived from the genus *Escherichia,* the genus *Bacillus,* the genus *Mycobacterium,* the genus *Zymomonas,* or the genus *Vibrio.* For example, PFK may be derived from *E.coli,* such as *E.coli* MG1655.

In an embodiment, the genetic modification that increases PFK activity in the recombinant microorganism is the introduction of a gene encoding a PFK having an activity belonging to EC 2.7.1.11.

In another embodiment, the genetic modification that increases PFK activity in the recombinant microorganism is the introduction of a gene encoding a polypeptide comprising an amino acid sequence having a sequence identity of about 90% or higher, about 95% or higher, or about 100% with the amino acid sequence of SEQ ID NO: 1. For example, the gene encoding PFK may comprise a nucleotide sequence having a sequence identity of about 90% or higher, about 95% or higher, or about 100% with the nucleotide sequence of SEQ ID NO: 2.

In the microorganism, the genetic modification may be one or more of increase in the expression of the gene encoding PFK and increase in the expression of the gene encoding PPK. The genetic modification may be an increase of the copy number of the gene encoding PFK or a modification of an expression regulatory sequence of the gene encoding PFK. Further, the genetic modification may be an increase of the copy number of the gene encoding PPK or a modification of an expression regulatory sequence of the gene encoding PFK. The increase of the copy number may be caused by introduction of the gene into a cell from the outside or by amplification of an endogenous gene.

The genetic modification may introduce the gene encoding PFK and the gene encoding PPK, for example, via a vehicle such as a vector. One or more of the gene encoding PFK and the gene encoding PPK may exist within or outside the chromosome. Furthermore, a plurality of genes (e.g., a plurality of copies) encoding PFK and/or genes encoding PPK may be introduced, for example, 2 or more, 5 or more, 10 or more, 30 or more, 50 or more, 100 or more, or 1000 or more of each of a gene encoding PFK or PPK.

The recombinant microorganism may belong to the genus *Komagataeibacter, Gluconacetobacter,* or *Enterobacter,* and may produce bacterial cellulose.

In one embodiment, the microorganism may belong to the genus *Komagataeibacter,* such as *K. xylinus* (also, referred to as *"G. xylinus*"), *K.rhaeticus, K. swingsii, K. kombuchae, K.nataicola,* or *K*. *sucrofermentans.* In some embodiments, the microorganism may have bacterial cellulose productivity. In some embodiments, the microorganism may not have endogenous PFK1. In some embodiments, the microorganism may not have an endogenous glycolytic pathway. Also, in some embodiments, the microorganism may not have endogenous PPK, or may not endogenously have PPK having higher catalytic activity for a reverse reaction than for a forward reaction in the reversible reaction catalyzed by the PPK enzyme. Further, the microorganism may not endogenously have PPK having high catalytic activity for conversion of NDP to NTP in a reaction using GDP, CDP, or UDP as a substrate, compared to using ADP, in the reverse reaction.

Another aspect of the invention provides a method of producing cellulose, the method including culturing a microorganism in a medium to produce cellulose; and collecting the cellulose from a culture.

In the inventive method, the microorganism may be any microorganism described herein.

In an embodiment the method comprises culturing a microorganism, such as a recombinant microorganism, including a genetic modification that increases a polyphosphate kinase activity in a medium to produce cellulose; and collecting the cellulose from a culture. In another embodiment the method comprises culturing a microorganism, such as a recombinant microorganism, including a genetic modification that increases a polyphosphate kinase activity and a genetic modification that increases expression of phosphofructose kinase or an activity thereof in a medium to produce cellulose; and collecting the cellulose from a culture.

The culturing may be performed in a medium containing a carbon source, for example, glucose. The medium used for culturing the microorganism may be any general medium suitable for host cell growth, such as a minimal or complex medium containing appropriate supplements. The suitable medium may be commercially available or prepared by a known preparation method.

The medium may be a medium that may satisfy the requirements of a particular microorganism depending on a selected product of culturing. The medium may be a medium including components selected from the group consisting of a carbon source, a nitrogen source, a salt, trace elements, and combinations thereof.

The medium may include ethanol or cellulose. The ethanol may be about 0.1%(v/v) to 5%(v/v), for example, about 0.3%(v/v) to 2.5%(v/v), about 0.3%(v/v) to 2.0%(v/v), about 0.3%(v/v) to 1.5%(v/v), about 0.3%(v/v) to 1.25%(v/v), about 0.3%(v/v) to 1.0%(v/v), about 0.3%(v/v) to 0.7%(v/v), or about 0.5%(v/v) to 3.0%(v/v) with respect to a volume of the medium. The cellulose may be about 0.5%(v/v) to 5%(w/v), about 0.5%(v/v) to 2.5%(w/v), about 0.5%(v/v) to 1.5%(w/v), or about 0.7%(v/v) to 1.25%(w/v) with respect to a weight of the medium. The cellulose may be carboxylated cellulose. The cellulose may be carboxy alkyl cellulose. The cellulose may be carboxy methyl cellulose (CMC). The CMC may be sodium CMC.

The culturing conditions may be appropriately controlled for the production of a selected product, for example, cellulose. The culturing may be performed under aerobic conditions for cell proliferation. The culturing may be performed by spinner culture or static culture without shaking. A density of the microorganism may be a density which gives enough space so as not to disturb production of cellulose.

The term "culture conditions", as used herein, mean conditions for culturing the microorganism. Such culture conditions may include, for example, a carbon source, a nitrogen source, or an oxygen condition utilized by the microorganism. The carbon source that may be utilized by the microorganism may include monosaccharides, disaccharides, or polysaccharides. The carbon source may include glucose, fructose, mannose, or galactose as an assimilable glucose. The nitrogen source may be an organic nitrogen compound or an inorganic nitrogen compound. The nitrogen source may be exemplified by amino acids, amides, amines, nitrates, or ammonium salts. An oxygen condition for culturing the microorganism may be an aerobic condition of a normal oxygen partial pressure or a low-oxygen condition including about 0.1% to about 10% oxygen in the atmosphere. A metabolic pathway may be modified in accordance with a carbon source or a nitrogen source that may be actually used by a microorganism.

The method may include collecting the cellulose from the culture. The separating may be, for example, collecting of a cellulose pellicle which is formed on the top of the medium. The cellulose pellicle may be collected by physically stripping off the cellulose pellicle or by removing the medium. The separating may be collecting of the cellulose pellicle while maintaining its shape without damage.

Still another aspect provides a method of producing the microorganism having enhanced cellulose productivity, the method including introducing the gene encoding polyphosphate kinase into a microorganism having cellulose productivity. The method may further include introducing the gene encoding PFK into the microorganism.

The introducing of the gene may be introducing of a vehicle including the gene into the microorganism. In the method, the genetic modification may include amplifying the gene, engineering a regulatory sequence of the gene, or engineering a sequence of the gene itself. The engineering may be insertion, substitution, conversion, or addition of a nucleotide.

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects.

Hereinafter, the present invention will be described in more detail with reference to Examples. However, these Examples are for illustrative purposes only, and the scope of the invention is not intended to be limited by these Examples.

### Example 1. Preparation of K.xylinus including phosphofructose kinase gene and Production of Cellulose

In this Example, *Komagataeibacter xylinus* DSM2325 was introduced with an exogenous PFK gene, and the microorganism introduced with the gene was cultured so that the microorganism was allowed to consume glucose and produce cellulose, thereby examining effects of the gene introduction on cellulose productivity.

### 1. Preparation of vector for over-expressing pfkA

The phosphofructose kinase (pfk) gene in *K.xylinus* was introduced by homologous recombination. A specific procedure is as follows.

An amplification product was obtained by PCR amplification using a pTSa-EX1 vector (SEQ ID NO: 9) as a template and a set of primers of SEQ ID NO: 5 and SEQ ID NO: 6 and a set of primers of SEQ ID NO: 7 and SEQ ID NO: 8. The amplification product was cloned by using an In-Fusion GD cloning kit (Takara) at the BamHI and Sail restriction sites of the pTSa-EX1 vector. The pTSa-EX1 vector is a shuttle vector which is replicable in both *E*. *coli* and *X*. *xylinus.*

In order to introduce pfkA by homologous recombination, an open reading frame (ORF) (SEQ ID NO: 2) of the pfkA gene was produced by PCR amplification using a genome DNA of *E*. *coli* K12 MG1655 as a template and a set of primers of SEQ ID NO: 3 and SEQ ID NO: 4 as primers. Fragments of the pfkA gene were cloned at the BamHI and Sail restriction enzyme sites of the pTSa-EX11 vector by using an In-Fusion GD cloning kit (Takara) to prepare vector pTSa-Ec.pfkA for over-expressing pfkA.

### 2. Preparation of vector for inserting E. coli pfkA gene

A tetA gene was amplified by PCR amplification using a pTSa-Ec.pfkA vector as a template and a set of primers of SEQ ID NO: 10 and SEQ ID NO: 11 as primers. The PCR product was cloned at an EcoRI restriction enzyme site of a pMSK+ vector (Genbank Accession No. KJ922019) by using an In-fusion GD cloning kit (Takara) to prepare a pTSK+ vector.

A homologous region of a site to which a pfkA gene was about to be inserted was amplified by PCR using a genome DNA of *K. xylinus* as a template and each of primer sets of SEQ ID NOS: 12 and 13, SEQ ID NOS: 14 and 15, and SEQ ID NOS: 16 and 17 as primers, and the amplification product was cloned at an EcoRI restriction enzyme site of a pTSK+ vector by using an In-fusion GD cloning kit (Takara) to prepare a pTSK-(del)2760 vector. 2760 gene encodes cytoplasmic NADPH-dependent glucose dehydrogenase.

A Ptac::Ec.pfkA gene was amplified by PCR amplification using the pTSa-Ec.pfkA vector as a template and a primer set of SEQ ID NO: 18 and SEQ ID NO: 19 as primers. The PCR product was cloned at an EcoRI restriction enzyme site of a pTSK-(del)2760 vector by using an In-fusion GD cloning kit (Takara) to prepare a pTSK-(del)2760-Ec.pfkA vector.

### 3. Introduction of phosphofructose kinase gene

In order to introduce the nucleotide sequence of SEQ ID NO: 2, which is a pkfA gene of *E.coli,* to *K.xylinus,* a cassette for inserting a Ptac::Ec.pfkA gene was amplified using the pTSK-(del)2760-Ec.pfkA vector as a template and a primer set of SEQ ID NO: 12 and SEQ ID NO: 17 as primers, and the amplification product was introduced into a *K. xylinus* strain by the following transformation method. A specific procedure is as follows.

The *K. xylinus* strain was spread on an HS medium (0.5% peptone, 0.5% yeast extract, 0.27% Na₂HPO₄, 0.15% citric acid, 2% glucose, and 1.5% bacto-agar) supplemented with 2% glucose, and then cultured at 30°C 3 days. The strain was inoculated in 5 ml of HS medium supplemented with 0.2%(v/v) of cellulase (sigma, Cellulase from *Trichoderma reesei* ATCC 26921), and then cultured at 30°C 2 days. A cell suspension thus cultured was inoculated in 100 ml of HS medium supplemented with 0.2%(v/v) of cellulase so that cell density (OD₆₀₀) was 0.04, and then the resultant was cultured at 30°C so that cell density was 0.4 to 0.7. The cultured strain was washed with 1 mM of HEPES buffer, washed three times with 15% glycerol, and re-suspended with 1 ml of 15% glycerol to prepare competent cells.

100 µl of the competent cells thus prepared was transferred to a 2 mm electro-cuvette, 3 µg of the Ptac::Ec.pfkA cassette prepared in the clause 2 was added thereto, and a vector was introduced into the competent cells by electroporation (2.4 kV, 200 Ω, 25 µF). The vector-introduced cells were re-suspended in 1 ml of HS medium containing 2% glucose and 0.1%(v/v) cellulase, transferred to a 14 ml round-bottom tube, and cultured at 30°C and 160 rpm for 16 hours. The cultured cells were spread on HS medium supplemented with 2% glucose, 1% ethanol, and 5 µg/ml of tetracycline, and cultured at 30°C for 4 days. Strains having a tetracycline resistance were selected to prepare pfk gene-over-expressing strains.

### 4. Glucose Consumption and Cellulose and Gluconate Productions

The designated *K.xylinus* strains were inoculated into 50 ml of HS medium supplemented with 5% glucose and 1% ethanol, and the resultant was cultured under stirring at 30°C at 230 rpm for 5 days. Then, glucose consumption and cellulose production were quantified. Glucose and gluconate were analyzed by using high performance liquid chromatography (HPLC) equipped with an Aminex HPX-87H column (Bio-Rad, USA). The cellulose production was quantified by measuring a weight after washing the cellulose solid produced in the flask with 0.1 N sodium hydroxide solution and distilled water, and freeze-drying the resultant. A gluconate yield was analyzed.

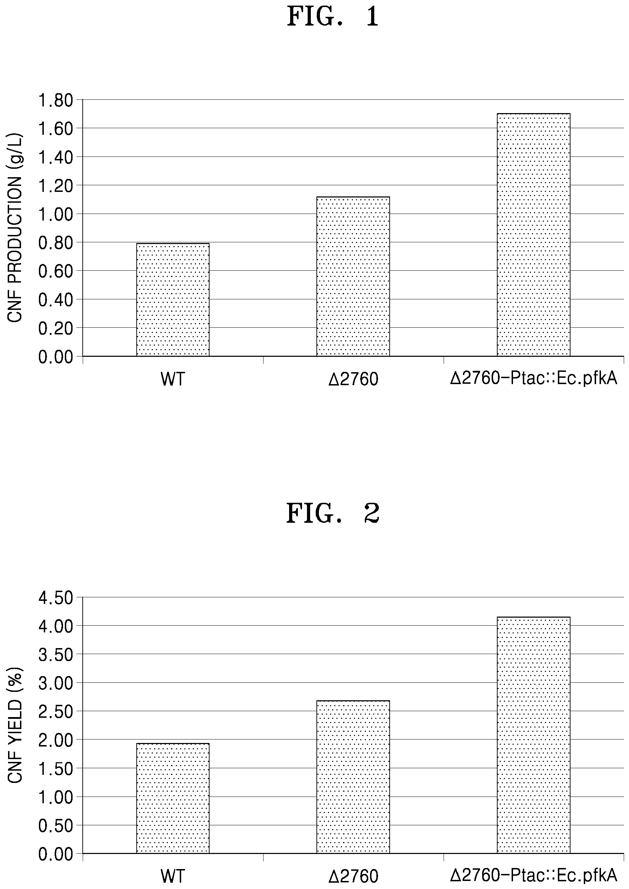

The results are shown in FIGS. 1 to 3. FIG. 1 shows CNF products from cultures which were obtained by culturing the *K.xylinus* strains. As shown in FIG. 1, when the pfkA gene was introduced into *K.xylinus,* the CNF production increased about 115% with respect to a wild-type strain. Table 1 illustrates the data shown in FIGS. 1 and 2.

**[Table 1]**

| | Glucose consumption (g/L) | Gluconate production (g/L) | CNF (g/L) | Gluconate yield (%) | CNF yield (%) |
|---|---|---|---|---|---|
| WT | 40.17 | 29.11 | 0.79 | 72.46 | 1.96 |
| Δ2760 | 41.65 | 31.71 | 1.12 | 76.13 | 2.68 |
| Δ2760-Ptac::Ec.pfkA | 40.76 | 29.85 | 1.70 | 73.24 | 4.18 |

FIG. 2 shows the yield of cellulose nanofibers (CNFs) obtained from the cultures prepared by culturing the *K.xylinus* strains. As shown in FIG. 2, when the pfkA gene was introduced into *K.xylinus,* the CNF yield increased about 113% with respect to a wild-type strain.

Also, the wild-type and recombinant strains were spread on HSD medium plates (5 g/L yeast extract, 5 g/L bacto peptone, 2.7 g/L Na₂HPO₄, 1.15 g/L citric acid, and 20 g/L glucose) containing 20 g/L agar, and cultured at 30°C for 3 days.

Starter fermentation was performed by adding 100 mL of HSD medium in a 250 mL flask, inoculating 3 loops of the microorganism, and culturing the resultant at 30°C at 150 rpm for 20 hours.

Main fermentation was performed by using a 1.5 L bench-type fermentor (GX2-series, Biotron) system, a baffle was removed, and a stirring environment with enhanced vertical movement was formed by using a pitch-type impeller and a microsparger.

Operation conditions included an initial volume of 0.7 L, a temperature of 30°C, pH 5.0 (adjusted by using a neutralizing agent 3 N KOH (aq)), a stirring rate of 150 rpm, an airflow amount of 0.7 L/min, a medium, which was HS medium supplemented with 40 g/L glucose, and inoculation at a rate of 14%(v/v).

In the CMC-added environment, fermentation evaluation included adding Na_CMC 1.0%(w/v) to the same HS medium and changing the stirring rate to 250 rpm from the conditions described above. A CNF quantity was measured based on a weight after pre-treating the collected fermentation solution, that is, washing the collected fermentation solution with a 0.1 N NaOH (aq) solution at 90°C for 2 hours.

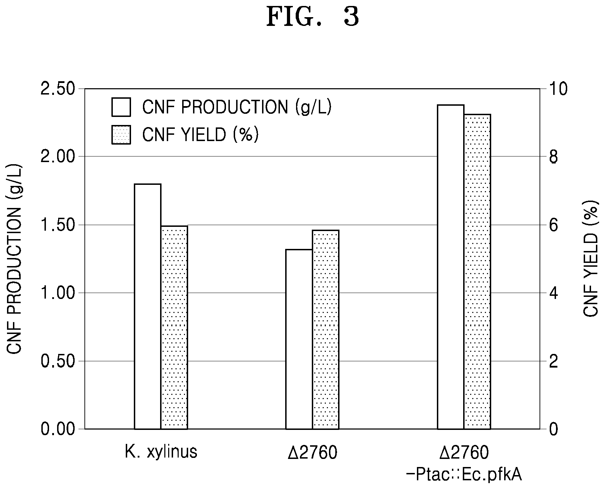

FIG. 3 shows CNF production and yield when the *K.xylinus* strains were cultured by fermentation. As shown in FIG. 3, when the pfkA gene was introduced into *K.xylinus,* CNF production increased about 32%, and the CNF yields increased about 55%, as compared with those of the control group. The yield is a percentage of the CNF weight produced with respect to a weight of glucose used. Table 2 illustrates the data shown in FIG. 3.

**[Table 2]**

| CMC free fermentation | Glucose consumption (g/L) | CNF production (g/L) | CNF yield (%) |
|---|---|---|---|
| WT | 29.70 | 1.80 | 5.95 |
| Δ2760 | 22.50 | 1.32 | 5.85 |
| Δ2760-Ptac::Ec.pfkA | 25.20 | 2.38 | 9.25 |

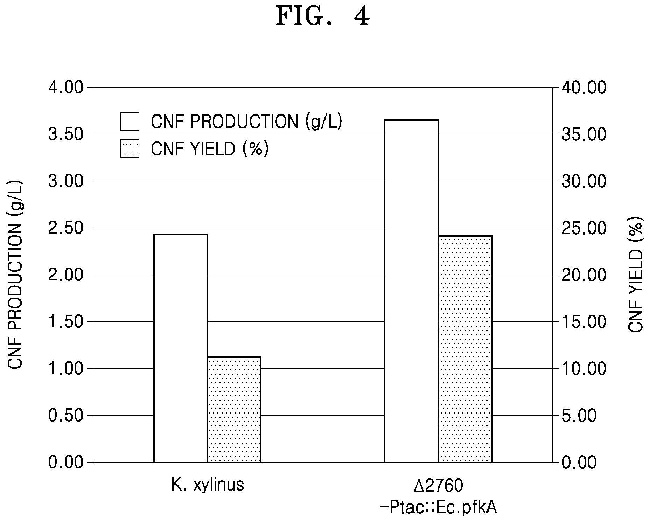

FIG. 4 shows CNF production and yield when the *K.xylinus* strains were fermented with CMC. As shown in FIG. 4, when the pfk gene was introduced into *K.xylinus,* the CNF production increased about 50%, and the CNF yields increased about 116%, as compared with those of the control group. Table 3 illustrates the data shown in FIG. 4.

**[Table 3]**

| CMC added fermentation | Glucose consumption (g/L) | CNF production (g/L) | CNF yield (%) |
|---|---|---|---|
| WT | 21.7 | 2.43 | 11.18 |
| Δ2760-Ptac::Ec.pfkA | 15.1 | 3.65 | 24.15 |

This indicates that the introduced exogenous pfkA phosphorylated fructose-6-phosphate of the strain into fructose-1,6-bisphosphate, and thus enhanced the glycolysis and influenced cellulose production.

### Example 2. Preparation of K.xylinus including polyphosphate kinase gene and Production of Cellulose

In this Example, the SK3 strain, which is a PFK gene-containing recombinant *K.xylinus* strain prepared in the clause 3 of Example 1, was introduced with an exogenous PPK gene, and the microorganism introduced with the gene was cultured so that the microorganism was allowed to consume glucose and produce cellulose, thereby examining effects of the gene introduction on cellulose productivity.

### 1. Preparation of vector

Vectors used in this Example were prepared as follows.

A pMKO vector was prepared as follows. gapA promoter and rrnB terminator regions were amplified by using a pTSa-EX2 vector (SEQ ID NO: 20) as a template and a set of primers of SEQ ID NO: 21 and SEQ ID NO: 23 and a set of primers of SEQ ID NO: 26 and SEQ ID NO: 22. kan^{R} gene was amplified by using a pK19 mob-sacB vector (ATCC® 87098™) as a template and a set of primers of SEQ ID NO: 24 and SEQ ID NO: 25. Each of the PCR products was cloned by using an In-Fusion GD cloning kit (Takara) at the BamHI restriction site of a pUC19 vector (TAKARA) to prepare a pMKO vector.

A pMcodBA vector was prepared as follows. codBA gene (SEQ ID NO: 50) was amplified by using gDNA of *E*. *coli* as a template and a set of primers of SEQ ID NO: 27 and SEQ ID NO: 28. codBA gene is a gene needed to remove a marker gene used in the preparation of the strain and is a gene for negative selection. This PCR product was cloned by using an In-Fusion GD cloning kit (Takara) at the BgIII restriction site of the pMKO vector to prepare a pMcodBA vector.

A pMCT vector was prepared as follows. tac promoter and rrnB terminator regions were amplified by using a pTSa-EX11 vector (SEQ ID NO: 20) as a template and a set of primers of SEQ ID NO: 29 and SEQ ID NO: 30 and a set of primers of SEQ ID NO: 31 and SEQ ID NO: 32. Further, codBA and kan^{R} genes were amplified by using a pMcodBA vector as a template and a set of primers of SEQ ID NO: 33 and SEQ ID NO: 22. Each of the PCR products was cloned by using an In-Fusion GD cloning kit (Takara) at the BamHI restriction site of a pUC19 vector to prepare a pMCT vector.

A pMCT-(del)zwf vector was prepared as follows. Upstream and downstream regions of zwf gene were amplified by using gDNA of *K. xylinus* as a template and a set of primers of SEQ ID NO: 34 and SEQ ID NO: 35 and a set of primers of SEQ ID NO: 36 and SEQ ID NO: 37. Each of the PCR products was cloned by using an In-Fusion GD cloning kit (Takara) at the BamHI restriction site of a pMCT vector to prepare a pMCT-(del)zwf vector.

To prepare a vector for inserting polyphosphate kinase (PPK) gene, *Silicibacter pomeroyi* PPK3 gene (SEQ ID NO: 45), *Silicibacter lacuscaerulensi* PPK2 (SEQ ID NO: 47) and *Rhodobacterales bacterium* PPK2 gene (SEQ ID NO: 49) were synthesized (pUC57-Sp.ppk / Sl.ppk / Rb.ppk). The PPK3, PPK2 and PPK2 encode amino acid sequences of SEQ ID NOS: 44, 46, and 48, respectively. Each of the ppk genes was amplified by using a set of primers of SEQ ID NO: 38 and SEQ ID NO: 39, a set of primers of SEQ ID NO: 40 and SEQ ID NO: 41, and a set of primers of SEQ ID NO: 42 and SEQ ID NO: 43, and each resulting product was cloned by using an In-Fusion GD cloning kit (Takara) at the BgIII restriction site of the pMCT-(del)zwf vector to prepare a pMCT-(del)zwf_Sp.ppk vector, a pMCT-(del)zwf_Sl.ppk vector, and a pMCT-(del)zwf_Rb.ppk vector, respectively.

### 2. Transformation

In order to introduce *Silicibacter pomeroyi* PPK3 (Sp ppk), *Silicibacter lacuscaerulensi* PPK2 (SI ppk) and *Rhodobacterales bacterium* PPK2 (Rb ppk) genes, pMCT-(del)zwf_Sp.ppk, pMCT-(del)zwf_Sl.ppk, and pMCT-(del)zwf_Rb.ppk were transformed into the SK3 strain which is a PFK gene-containing recombinant *K.xylinus* strain prepared in the clause 3 of Example 1 by the following transformation method to prepare SK3-(del)zwf_Sp.ppk, Sl.ppk, and Rb.ppk strains, respectively. A specific transformation procedure is as follows.

*K.xylinus* strain was spread on 2% glucose-supplemented HS medium containing 0.5% peptone, 0.5% yeast extract, 0.27% Na₂HPO₄, 0.15% citric acid, 2% glucose, and 1.5% bacto-agar, and cultured at 30°C for 3 days. The strain was inoculated in 5 ml of HS medium supplemented with 0.2% cellulase (sigma), and then cultured at 30°C for 2 days. A cell suspension thus cultured was inoculated in 100 ml of HS medium supplemented with 0.2% cellulase so that cell density (OD₆₀₀) was 0.04, and then the resultant was cultured at 30°C so that cell density was 0.4 to 0.7. The cultured strain was washed with 1 mM of HEPES buffer, washed three times with 15% glycerol, and re-suspended with 1 ml of 15% glycerol to prepare competent cells.

*K. xylinus* DSM2325 M9 strain was spread on 2% glucose-supplemented HS plate, and then cultured at 30°C for 3 days. The strain thus cultured was transferred to a 50 ml-falcon tube by using sterile water, followed by vortexing for 2 minutes. 1% cellulase (sigma, Cellulase from *Trichoderma reesei* ATCC 26921) was added thereto, and reaction was allowed at 30°C and 160 rpm for 2 hours. Thereafter, the cultured strain was washed with 1 mM of HEPES buffer, washed three times with 15% glycerol, and re-suspended with 1 ml of 15% glycerol. 100 µl of the competent cells thus prepared were transferred to a 2-mm electro-cuvette, 3 µg of the plasmid was added thereto, and transformation was performed by electroporation (3.0 kV, 250 Ω, 25 µF). The cells were re-suspended in 1 ml of HS medium (2% glucose), transferred to a 14-ml round-bottom tube, and cultured at 30°C and 230 rpm for 16 hours. The cultured cells were spread on an HS plate supplemented with 2% glucose, 1% ethanol, and 5 µg/ml of tetracycline or 50 µg/ml of kanamycin, and cultured at 30°C for 5 days.

### 3. CNF production

The *K.xylinus* strains introduced with respective vectors were streaked on HS plate supplemented with 2% glucose, 1% ethanol, and 5 µg/ml of tetracycline or 50 µg/ml of kanamycin, and cultured at 30°C for 5 days. Then, the strains thus cultured were inoculated in 25 ml of HS medium supplemented with 5% glucose and 1% ethanol, and then cultured at 30°C, 230 rpm for 6 days. CNF thus produced was washed with 0.1N NaOH and distilled water at 60°C, and then freeze-dried to remove H₂O therefrom, followed by weighing. Glucose and gluconate were analyzed by HPLC.

CNF productions of the SK3 strains introduced with the respective ppk genes were compared, and as a result, the SK3 strains introduced with the respective ppk genes showed increased CNF productions, as compared with SK3 strain.

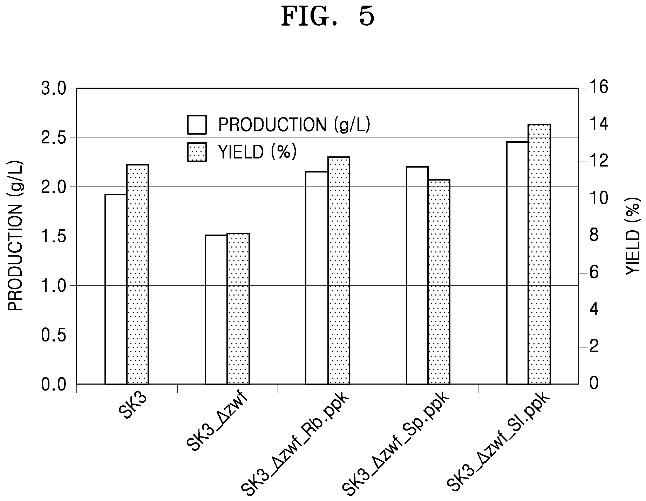

FIG. 5 shows results of comparing CNF productions of the SK3 strains which were introduced with the respective ppk genes. Table 4 shows the results of FIG. 5.

**[Table 4]**

| Strain | Production (g/L) | | | CNF yield (%) | |
|---|---|---|---|---|---|
| | Glucose consumed | Gluconate | CNF | CNF | Gluconate |
| SK3 | 16.20 | 10.21 | 1.92 | 11.85 | 63.05 |
| SK3_Δzwf | 18.55 | 7.92 | 1.51 | 8.14 | 42.70 |
| SK3_Δzwf_Rb ppk | 17.49 | 8.09 | 2.15 | 12.29 | 46.25 |
| SK3_Δzwf_Sp ppk | 19.93 | 3.95 | 2.21 | 11.09 | 19.84 |
| SK3_Δzwf_Sl ppk | 17.46 | 9.70 | 2.45 | 14.03 | 55.53 |

As shown in Table 4, the CNF production increased about 62.3%, and the CNF yield increased about 72.4% in SK3_Δzwf_Sl ppk, as compared with SK3_Δzwf, indicating that introduced exogenous PPK enhanced supply of cofactors including ATP, GTP, UTP, CTP, or a combination thereof and influenced cellulose production.

The use of the terms "a" and "an" and "the" and "at least one" and similar referents in the context of describing the invention (especially in the context of the following claims) are to be construed to cover both the singular and the plural, unless otherwise indicated herein or clearly contradicted by context. The use of the term "at least one" followed by a list of one or more items (for example, "at least one of A and B") is to be construed to mean one item selected from the listed items (A or B) or any combination of two or more of the listed items (A and B), unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

## Claims

1. A microorganism of the genus *Komagataeibacter* comprising a genetic modification that increases expression or activity of polyphosphate kinase (PPK).

2. The microorganism of claim 1, wherein the genetic modification increases expression of a gene encoding the polyphosphate kinase.

3. The microorganism of claim 1 or 2, wherein the genetic modification is an increase in the copy number of a gene encoding the polyphosphate kinase or a modification of an expression regulatory sequence of the gene encoding the polyphosphate kinase.

4. The microorganism of any one of claims 1 to 3, wherein the polyphosphate kinase belongs to EC 2.7.4.1.

5. The microorganism of any one of claims 1 to 4, wherein the polyphosphate kinase catalyzes both the forward and reverse reaction of converting NTP + (phosphate)n to NDP + (phosphate)n+1, and has higher catalytic activity for the reverse reaction than for the forward reaction, wherein preferably the polyphosphate kinase has higher catalytic activity for conversion of NDP to NTP in a reaction using GDP, CDP, or UDP as a substrate, compared to using ADP.

6. The microorganism of any one of claims 1 to 5, wherein the polyphosphate kinase is a polypeptide having a sequence identity of 85% or more with an amino acid sequence of SEQ ID NO: 44, 46, or 48.

7. The microorganism of any one of claims 1 to 6, wherein the polyphosphate kinase is a *Silicibacter* polyphosphate kinase or a *Rhodobacterales* polyphosphate kinase.

8. The microorganism of any one of claims 1 to 7, wherein the microorganism has enhanced cellulose productivity as compared to a microorganism of the same type without the genetic modification that increases expression or activity of the polyphosphate kinase (PPK).

9. The microorganism of any one of claims 1 to 8, further comprising a genetic modification that increases expression or activity of phosphofructose kinase (PFK), wherein preferably the genetic modification is an increase of the copy number of a gene encoding the phosphofructose kinase or a modification of an expression regulatory sequence of the gene encoding the phosphofructose kinase.

10. The microorganism of claim 9, wherein the phosphofructose kinase belongs to EC 2.7.1.11, and/or wherein the phosphofructose kinase is a polypeptide having a sequence identity of 90% or more with an amino acid sequence of SEQ ID NO: 1, and/or wherein the phosphofructose kinase is a *Escherichia* phosphofructose kinase, *Bacillus* phosphofructose kinase, *Mycobacterium* phosphofructose kinase, *Zymomonas* phosphofructose kinase, or *Vibrio* phosphofructose kinase.

11. The microorganism of any one of claims 1 to 10, wherein the microorganism is *Komagataeibacter xylinus.*

12. A method of producing cellulose, the method comprising:
culturing the microorganism of any one of claims 1 to 11 in a medium to produce cellulose; and
collecting the cellulose from a culture.

13. The method of claim 12, wherein the medium comprises ethanol or exogenous cellulose, wherein the cellulose preferably is carboxylated cellulose, and wherein most preferably the carboxylated cellulose is carboxy alkyl cellulose.

14. A method of producing a recombinant microorganism having enhanced cellulose productivity, the method comprising introducing a gene encoding polyphosphate kinase into a microorganism.

15. The method of claim 14, further comprising introducing a gene encoding phosphofructose kinase into the microorganism.


<110>    Samsung Electronics Co., Ltd.

<120>    Recombinant Microorganism of Genus Komagataeibacter,
         Method of Producing Cellulose by Using the Same,
         and Method of Producing the Microorganism

<130>    EP117607FZTRpau

<140>    not yet assigned
<141>    herewith

<150>    10-2017-0161834
<151>    2017-11-29

<150>    10-2017-0098071
<151>    2017-08-02

<160>    50

<170>    KopatentIn 2.0

<210>    1
<211>    320
<212>    PRT
<213>    E.coli

<400>    1
Met Ile Lys Lys Ile Gly Val Leu Thr Ser Gly Gly Asp Ala Pro Gly
  1               5                  10                  15

Met Asn Ala Ala Ile Arg Gly Val Val Arg Ser Ala Leu Thr Glu Gly
             20                  25                  30

Leu Glu Val Met Gly Ile Tyr Asp Gly Tyr Leu Gly Leu Tyr Glu Asp
         35                  40                  45

Arg Met Val Gln Leu Asp Arg Tyr Ser Val Ser Asp Met Ile Asn Arg
     50                  55                  60

Gly Gly Thr Phe Leu Gly Ser Ala Arg Phe Pro Glu Phe Arg Asp Glu
 65                  70                  75                  80

Asn Ile Arg Ala Val Ala Ile Glu Asn Leu Lys Lys Arg Gly Ile Asp
                 85                  90                  95

Ala Leu Val Val Ile Gly Gly Asp Gly Ser Tyr Met Gly Ala Met Arg
            100                 105                 110

Leu Thr Glu Met Gly Phe Pro Cys Ile Gly Leu Pro Gly Thr Ile Asp
        115                 120                 125

Asn Asp Ile Lys Gly Thr Asp Tyr Thr Ile Gly Phe Phe Thr Ala Leu
    130                 135                 140

Ser Thr Val Val Glu Ala Ile Asp Arg Leu Arg Asp Thr Ser Ser Ser
145                 150                 155                 160

His Gln Arg Ile Ser Val Val Glu Val Met Gly Arg Tyr Cys Gly Asp
                165                 170                 175

Leu Thr Leu Ala Ala Ala Ile Ala Gly Gly Cys Glu Phe Val Val Val
            180                 185                 190

Pro Glu Val Glu Phe Ser Arg Glu Asp Leu Val Asn Glu Ile Lys Ala
        195                 200                 205

Gly Ile Ala Lys Gly Lys Lys His Ala Ile Val Ala Ile Thr Glu His
    210                 215                 220

Met Cys Asp Val Asp Glu Leu Ala His Phe Ile Glu Lys Glu Thr Gly
225                 230                 235                 240

Arg Glu Thr Arg Ala Thr Val Leu Gly His Ile Gln Arg Gly Gly Ser
                245                 250                 255

Pro Val Pro Tyr Asp Arg Ile Leu Ala Ser Arg Met Gly Ala Tyr Ala
            260                 265                 270

Ile Asp Leu Leu Leu Ala Gly Tyr Gly Gly Arg Cys Val Gly Ile Gln
        275                 280                 285

Asn Glu Gln Leu Val His His Asp Ile Ile Asp Ala Ile Glu Asn Met
    290                 295                 300

Lys Arg Pro Phe Lys Gly Asp Trp Leu Asp Cys Ala Lys Lys Leu Tyr
305                 310                 315                 320

<210> 2
<211> 963
<212> DNA
<213> E.coli

<400> 2
atgattaaga aaatcggtgt gttgacaagc ggcggtgatg cgccaggcat gaacgccgca 60

attcgcgggg ttgttcgttc tgcgctgaca gaaggtctgg aagtaatggg tatttatgac 120

ggctatctgg gtctgtatga agaccgtatg gtacagctag accgttacag cgtgtctgac 180

atgatcaacc gtggcggtac gttcctcggt tctgcgcgtt tcccggaatt ccgcgacgag 240

aacatccgcg ccgtggctat cgaaaacctg aaaaaacgtg gtatcgacgc gctggtggtt 300

atcggcggtg acggttccta catgggtgca atgcgtctga ccgaaatggg cttcccgtgc 360

atcggtctgc cgggcactat cgacaacgac atcaaaggca ctgactacac tatcggtttc 420

ttcactgcgc tgagcaccgt tgtagaagcg atcgaccgtc tgcgtgacac ctcttcttct 480

caccagcgta tttccgtggt ggaagtgatg ggccgttatt gtggagatct gacgttggct 540

gcggccattg ccggtggctg tgaattcgtt gtggttccgg aagttgaatt cagccgtgaa 600

gacctggtaa acgaaatcaa agcgggtatc gcgaaaggta aaaaacacgc gatcgtggcg 660

attaccgaac atatgtgtga tgttgacgaa ctggcgcatt tcatcgagaa agaaaccggt 720

cgtgaaaccc gcgcaactgt gctgggccac atccagcgcg gtggttctcc ggtgccttac 780

gaccgtattc tggcttcccg tatgggcgct tacgctatcg atctgctgct ggcaggttac 840

ggcggtcgtt gtgtaggtat ccagaacgaa cagctggttc accacgacat catcgacgct 900

atcgaaaaca tgaagcgtcc gttcaaaggt gactggctgg actgcgcgaa aaaactgtat 960

taa                                                                    963

<210>    3
<211>    39
<212>    DNA
<213>    Artificial Sequence

<220>
<223>    PFKA primer

<400>    3
cgtacccggg gatccatgat taagaaaatc ggtgtgttg                             39

<210>    4
<211>    39
<212>    DNA
<213>    Artificial Sequence

<220>
<223>    PFKA primer

<400>    4
gactctagag gatccttaat acagtttttt cgcgcagtc                             39

<210>    5
<211>    33
<212>    DNA
<213>    Artificial Sequence

<220>
<223>    F1 forward primer

<400>    5
cggcgtagag gatcaggagc ttatcgactg cac                                   33

<210>    6
<211>    28
<212>    DNA
<213>    Artificial Sequence

<220>
<223>    F1 reverse primer

<400>    6
ccggcgtaga gaatccacag gacgggtg                                         28

<210>    7
<211>    27
<212>    DNA
<213>    Artificial Sequence

<220>
<223>    F2 forward primer

<400>    7
ctgtggattc tctacgccgg acgcatc                                          27


<210>    8
<211>    29
<212>    DNA
<213>    Artificial Sequence

<220>
<223>    F2 reverse primer


<400>    8
aagggcatcg gtcgtcgctc tcccttatg                                        29


<210>    9
<211>    3576
<212>    DNA
<213>    Artificial Sequence

<220>
<223>    pTSa-EX1 vector


<400>    9
gaattcagcc agcaagacag cgatagaggg tagttatcca cgtgaaaccg ctaatgcccc      60

gcaaagcctt gattcacggg gctttccggc ccgctccaaa aactatccac gtgaaatcgc     120

taatcagggt acgtgaaatc gctaatcgga gtacgtgaaa tcgctaataa ggtcacgtga     180

aatcgctaat caaaaaggca cgtgagaacg ctaatagccc tttcagatca acagcttgca     240

aacacccctc gctccggcaa gtagttacag caagtagtat gttcaattag cttttcaatt     300

atgaatatat atatcaatta ttggtcgccc ttggcttgtg gacaatgcgc tacgcgcacc     360

ggctccgccc gtggacaacc gcaagcggtt gcccaccgtc gagcgccagc gcctttgccc     420

acaacccggc ggccggccgc aacagatcgt tttataaatt tttttttttg aaaaagaaaa     480

agcccgaaag gcggcaacct ctcgggcttc tggatttccg atcacctgta agtcggacgt     540

tccgatcacc tgtaacgatg cgtccggcgt agaggatccg gagcttatcg actgcacggt     600

gcaccaatgc ttctggcgtc aggcagccat cggaagctgt ggtatggctg tgcaggtcgt     660

aaatcactgc ataattcgtg tcgctcaagg cgcactcccg ttctggataa tgttttttgc     720

gccgacatca taacggttct ggcaaatatt ctgaaatgag ctgttgacaa ttaatcatcg     780

gctcgtataa tgtgtggaat tgtgagcgga taacaatttc acacaggacg gagctattga     840

ttgggtaccg agctcgaatt cgtacccggg gatcctctag agtcgacctg caggcatgca     900

agcttggctg ttttggcgga tgagagaaga ttttcagcct gatacagatt aaatcagaac     960

gcagaagcgg tctgataaaa cagaatttgc ctggcggcag tagcgcggtg gtcccacctg    1020

accccatgcc gaactcagaa gtgaaacgcc gtagcgccga tggtagtgtg gggtctcccc    1080

atgcgagagt agggaactgc caggcatcaa ataaaacgaa aggctcagtc gaaagactgg 1140

gcctttcgtt ttatctgttg tttgtcggtg aacgctctcc tgagtaggac aaatccgccg 1200

ggagcggatt tgaacgttgc gaagcaacgg cccggagggt ggcgggcagg acgcccgcca 1260

taaactgcca ggcatcaaat taagcagaag gccatcctga cggatggcct ttttgcaaga 1320

acatgtgagc acttccgctt cctcgctcac tgactcgctg cgctcggtcg ttcggctgcg 1380

gcgagcggta tcagctcact caaaggcggt aatacggtta tccacagaat caggggataa 1440

cgcaggaaag aacatgtgag caaaaggcca gcaaaaggcc aggaaccgta aaaaggccgc 1500

gttgctggcg tttttccata ggctccgccc ccctgacgag catcacaaaa atcgacgctc 1560

aagtcagagg tggcgaaacc cgacaggact ataaagatac caggcgtttc cccctggaag 1620

ctccctcgtg cgctctcctg ttccgaccct gccgcttacc ggatacctgt ccgcctttct 1680

cccttcggga agcgtggcgc tttctcatag ctcacgctgt aggtatctca gttcggtgta 1740

ggtcgttcgc tccaagctgg gctgtgtgca cgaacccccc gttcagcccg accgctgcgc 1800

cttatccggt aactatcgtc ttgagtccaa cccggtaaga cacgacttat cgccactggc 1860

agcagccact ggtaacagga ttagcagagc gaggtatgta ggcggtgcta cagagttctt 1920

gaagtggtgg cctaactacg gctacactag aagaacagca tttggtatct gcgctctgct 1980

gaagccagtt accttcggaa aaagagttgg tagctcttga tccggcaaac aaaccaccgc 2040

tggtagcggt ggtttttttg tttgcaagca gcagattacg cgcagaaaaa aaggatctca 2100

agaagatcct ttgatctttt ctacggggtc tgacgctcag tggaacgaaa actcacgtta 2160

attctcatgt ttgacagctt atcatcgata agctttaatg cggtagttta tcacagttaa 2220

attgctaacg cagtcaggca ccgtgtatga aatctaacaa tgcgctcatc gtcatcctcg 2280

gcaccgtcac cctggatgct gtaggcatag gcttggttat gccggtactg ccgggcctct 2340

tgcgggatat cgtccattcc gacagcatcg ccagtcacta tggcgtgctg ctagcgctat 2400

atgcgttgat gcaatttcta tgcgcacccg ttctcggagc actgtccgac cgctttggcc 2460

gccgcccagt cctgctcgct tcgctacttg gagccactat cgactacgcg atcatggcga 2520

ccacacccgt cctgtggatc ctctacgccg gacgcatcgt ggccggcatc accggcgcca 2580

caggtgcggt tgctggcgcc tatatcgccg acatcaccga tggggaagat cgggctcgcc 2640

acttcgggct catgagcgct tgtttcggcg tgggtatggt ggcaggcccc gtggccgggg 2700

gactgttggg cgccatctcc ttgcatgcac cattccttgc ggcggcggtg ctcaacggcc 2760

tcaacctact actgggctgc ttcctaatgc aggagtcgca taagggagag cgtcgaccga 2820

tgcccttgag agccttcaac ccagtcagct ccttccggtg ggcgcggggc atgactatcg 2880

tcgccgcact tatgactgtc ttctttatca tgcaactcgt aggacaggtg ccggcagcgc 2940

tctgggtcat tttcggcgag gaccgctttc gctggagcgc gacgatgatc ggcctgtcgc 3000

ttgcggtatt cggaatcttg cacgccctcg ctcaagcctt cgtcactggt cccgccacca 3060

aacgtttcgg cgagaagcag gccattatcg ccggcatggc ggccgacgcg ctgggctacg 3120

tcttgctggc gttcgcgacg cgaggctgga tggccttccc cattatgatt cttctcgctt 3180

ccggcggcat cgggatgccc gcgttgcagg ccatgctgtc caggcaggta gatgacgacc 3240

atcagggaca gcttcaagga tcgctcgcgg ctcttaccag cctaacttcg atcactggac 3300

cgctgatcgt cacggcgatt tatgccgcct cggcgagcac atggaacggg ttggcatgga 3360

ttgtaggcgc cgccctatac cttgtctgcc tccccgcgtt gcgtcgcggt gcatggagcc 3420

gggccacctc gacctgaatg gaagccggcg gcacctcgct aacggattca ccactccaag 3480

aattggagcc aatttttaag gcagttattg gtgcccttaa acgcctggtt gctacgcctg 3540

aataagtgat aataagcgga tgaatggcag aaattc 3576

<210> 10
<211> 40
<212> DNA
<213> Artificial Sequence

<220>
<223> primer

<400> 10
cttgatatcg aattcttctc atgtttgaca gcttatcatc 40

<210> 11
<211> 36
<212> DNA
<213> Artificial Sequence

<220>
<223> primer

<400> 11
gggctgcagg aattcgaatt tctgccattc atccgc 36

<210> 12
<211> 37
<212> DNA
<213> Artificial Sequence

<220>
<223> primer

<400> 12
cttgatatcg aattaggcct gtcatcgtct atatacg 37

<210> 13

<211>    42
<212>    DNA
<213>    Artificial Sequence

<220>
<223>    primer


<400>    13
cgtgttgttc gaattcgatg gatattcctc cagtatcatg tg                          42


<210>    14
<211>    32
<212>    DNA
<213>    Artificial Sequence

<220>
<223>    primer


<400>    14
catcgaattc gaacaacacg ccgatgtatg ac                                     32


<210>    15
<211>    40
<212>    DNA
<213>    Artificial Sequence

<220>
<223>    primer


<400>    15
acatgagaag aattgacaga tccggtcagt tcacattatc                             40


<210>    16
<211>    39
<212>    DNA
<213>    Artificial Sequence

<220>
<223>    primer


<400>    16
cagaaattcg aattgcgatc atcaccaacc aggaaattc                              39


<210>    17
<211>    37
<212>    DNA
<213>    Artificial Sequence

<220>
<223>    primer


<400>    17
gggctgcagg aattgggtat ttcaggcggc agtaaag                                37

<210>    18
<211>    40
<212>    DNA
<213>    Artificial Sequence

<220>
<223>    primer

<400>    18
cttgatatcg aattcttctc atgtttgaca gcttatcatc                              40

<210>    19
<211>    36
<212>    DNA
<213>    Artificial Sequence

<220>
<223>    primer

<400>    19
gggctgcagg aattcgaatt tctgccattc atccgc                                  36

<210>    20
<211>    3810
<212>    DNA
<213>    Artificial Sequence

<220>
<223>    pTSa-EX2 vector

<400>    20
gaattcagcc agcaagacag cgatagaggg tagttatcca cgtgaaaccg ctaatgcccc        60

gcaaagcctt gattcacggg gctttccggc ccgctccaaa aactatccac gtgaaatcgc       120

taatcagggt acgtgaaatc gctaatcgga gtacgtgaaa tcgctaataa ggtcacgtga       180

aatcgctaat caaaaaggca cgtgagaacg ctaatagccc tttcagatca acagcttgca       240

aacacccctc gctccggcaa gtagttacag caagtagtat gttcaattag cttttcaatt       300

atgaatatat atatcaatta ttggtcgccc ttggcttgtg gacaatgcgc tacgcgcacc       360

ggctccgccc gtggacaacc gcaagcggtt gcccaccgtc gagcgccagc gcctttgccc       420

acaacccggc ggccggccgc aacagatcgt tttataaatt tttttttttg aaaaagaaaa       480

agcccgaaag gcggcaacct ctcgggcttc tggatttccg atcacctgta agtcggacga       540

acttcggcgg cgcccgagcg tgaacagcac gggctgacca acctgtgcgc gcggggcggt       600

tacgtgctgg ccgaagccga aggcgcgcga caggtcacgc tgatcgccac gggacatgag       660

gccatactgg cactggcggc gcgcaaactg ctgcgggacg cgggggttgc ggcggctgtc       720

gtctcccttc catgctggga actgttcgcc gtgcaaaaaa tgacgtatcg tgccgccgtg       780

ctgggaacgg caccccggat cgggatcgag gccgcttcag ggtttggatg ggaacgatgg 840
cttggaacag gcgggctgtt tgtcggtatt gacggattcg gggcgtctta cgcccccgac 900
cggccagaca gccctgccgg catcacgccg gaacggatct gccacgacgc attgcggctg 960
gtccgccccc atgccgacgc cctggttgaa accgcgggag gaaacggcgc gccgcccggg 1020
atggcatcgg tcgatgccag tgtgtgaaat gtcagacctt acggagaaaa taagaaaagg 1080
acgagctatt gattcgtacc cggggatcct ctagagtcga cctgcaggca tgcaagcttg 1140
gctgttttgg cggatgagag aagattttca gcctgataca gattaaatca gaacgcagaa 1200
gcggtctgat aaaacagaat ttgcctggcg gcagtagcgc ggtggtccca cctgacccca 1260
tgccgaactc agaagtgaaa cgccgtagcg ccgatggtag tgtggggtct ccccatgcga 1320
gagtagggaa ctgccaggca tcaaataaaa cgaaaggctc agtcgaaaga ctgggccttt 1380
cgttttatct gttgtttgtc ggtgaacgct ctcctgagta ggacaaatcc gccgggagcg 1440
gatttgaacg ttgcgaagca acggcccgga gggtggcggg caggacgccc gccataaact 1500
gccaggcatc aaattaagca gaaggccatc ctgacggatg gcctttttgc aagaacatgt 1560
gagcacttcc gcttcctcgc tcactgactc gctgcgctcg gtcgttcggc tgcggcgagc 1620
ggtatcagct cactcaaagg cggtaatacg gttatccaca gaatcagggg ataacgcagg 1680
aaagaacatg tgagcaaaag gccagcaaaa ggccaggaac cgtaaaaagg ccgcgttgct 1740
ggcgtttttc cataggctcc gcccccctga cgagcatcac aaaaatcgac gctcaagtca 1800
gaggtggcga aacccgacag gactataaag ataccaggcg tttccccctg gaagctccct 1860
cgtgcgctct cctgttccga ccctgccgct taccggatac ctgtccgcct ttctcccttc 1920
gggaagcgtg gcgctttctc atagctcacg ctgtaggtat ctcagttcgg tgtaggtcgt 1980
tcgctccaag ctgggctgtg tgcacgaacc ccccgttcag cccgaccgct gcgccttatc 2040
cggtaactat cgtcttgagt ccaacccggt aagacacgac ttatcgccac tggcagcagc 2100
cactggtaac aggattagca gagcgaggta tgtaggcggt gctacagagt tcttgaagtg 2160
gtggcctaac tacggctaca ctagaagaac agcatttggt atctgcgctc tgctgaagcc 2220
agttaccttc ggaaaaagag ttggtagctc ttgatccggc aaacaaacca ccgctggtag 2280
cggtggtttt tttgtttgca agcagcagat tacgcgcaga aaaaaaggat ctcaagaaga 2340
tcctttgatc ttttctacgg ggtctgacgc tcagtggaac gaaaactcac gttaattctc 2400
atgtttgaca gcttatcatc gataagcttt aatgcggtag tttatcacag ttaaattgct 2460
aacgcagtca ggcaccgtgt atgaaatcta acaatgcgct catcgtcatc ctcggcaccg 2520
tcaccctgga tgctgtaggc ataggcttgg ttatgccggt actgccgggc ctcttgcggg 2580
atatcgtcca ttccgacagc atcgccagtc actatggcgt gctgctagcg ctatatgcgt 2640
tgatgcaatt tctatgcgca cccgttctcg gagcactgtc cgaccgcttt ggccgccgcc 2700

cagtcctgct cgcttcgcta cttggagcca ctatcgacta cgcgatcatg gcgaccacac 2760

ccgtcctgtg gatcctctac gccggacgca tcgtggccgg catcaccggc gccacaggtg 2820

cggttgctgg cgcctatatc gccgacatca ccgatgggga agatcgggct cgccacttcg 2880

ggctcatgag cgcttgtttc ggcgtgggta tggtggcagg ccccgtggcc gggggactgt 2940

tgggcgccat ctccttgcat gcaccattcc ttgcggcggc ggtgctcaac ggcctcaacc 3000

tactactggg ctgcttccta atgcaggagt cgcataaggg agagcgtcga ccgatgccct 3060

tgagagcctt caacccagtc agctccttcc ggtgggcgcg gggcatgact atcgtcgccg 3120

cacttatgac tgtcttcttt atcatgcaac tcgtaggaca ggtgccggca gcgctctggg 3180

tcattttcgg cgaggaccgc tttcgctgga gcgcgacgat gatcggcctg tcgcttgcgg 3240

tattcggaat cttgcacgcc ctcgctcaag ccttcgtcac tggtcccgcc accaaacgtt 3300

tcggcgagaa gcaggccatt atcgccggca tggcggccga cgcgctgggc tacgtcttgc 3360

tggcgttcgc gacgcgaggc tggatggcct tccccattat gattcttctc gcttccggcg 3420

gcatcgggat gcccgcgttg caggccatgc tgtccaggca ggtagatgac gaccatcagg 3480

gacagcttca aggatcgctc gcggctctta ccagcctaac ttcgatcact ggaccgctga 3540

tcgtcacggc gatttatgcc gcctcggcga gcacatggaa cgggttggca tggattgtag 3600

gcgccgccct ataccttgtc tgcctccccg cgttgcgtcg cggtgcatgg agccgggcca 3660

cctcgacctg aatggaagcc ggcggcacct cgctaacgga ttcaccactc caagaattgg 3720

agccaatttt taaggcagtt attggtgccc ttaaacgcct ggttgctacg cctgaataag 3780

tgataataag cggatgaatg gcagaaattc 3810

<210> 21
<211> 32
<212> DNA
<213> Artificial Sequence

<220>
<223> primer

<400> 21
gactctagag gatccaactt cggcggcgcc cg 32

<210> 22
<211> 33
<212> DNA
<213> Artificial Sequence

<220>
<223> primer

<400> 22

ggtacccggg gatccgcaaa aaggccatcc gtc 33

<210> 23
<211> 39
<212> DNA
<213> Artificial Sequence

<220>
<223> primer

<400> 23
aatattattg agatcttttc ttattttctc cgtaaggtc 39

<210> 24
<211> 33
<212> DNA
<213> Artificial Sequence

<220>
<223> primer

<400> 24
gaaaagatct caataatatt gaaaaaggaa gag 33

<210> 25
<211> 31
<212> DNA
<213> Artificial Sequence

<220>
<223> primer

<400> 25
ccgccaggca tcagaagaac tcgtcaagaa g 31

<210> 26
<211> 26
<212> DNA
<213> Artificial Sequence

<220>
<223> primer

<400> 26
gttcttctga tgcctggcgg cagtag 26

<210> 27
<211> 38
<212> DNA
<213> Artificial Sequence

<220>
<223> primer

<400>    27
aataagaaaa gatcaatgtc gcaagataac aactttag                              38


<210>    28
<211>    54
<212>    DNA
<213>    Artificial Sequence

<220>
<223>    primer


<400>    28
atattattga gatcaaaggt ctgacatttg atcatcaacg tttgtaatcg atgg            54


<210>    29
<211>    33
<212>    DNA
<213>    Artificial Sequence

<220>
<223>    primer


<400>    29
cgactctaga ggatcccggc gtagaggatc agg                                   33


<210>    30
<211>    46
<212>    DNA
<213>    Artificial Sequence

<220>
<223>    primer


<400>    30
ccgccaggca acctagatct ggatcagctc ggtacccaat caatag                     46


<210>    31
<211>    26
<212>    DNA
<213>    Artificial Sequence

<220>
<223>    primer


<400>    31
agatctaggt tgcctggcgg cagtag                                           26


<210>    32
<211>    34
<212>    DNA
<213>    Artificial Sequence

<220>

<223>    primer

<400>    32
gggcgccgcc gaagttgcaa aaaggccatc cgtc                                    34

<210>    33
<211>    26
<212>    DNA
<213>    Artificial Sequence

<220>
<223>    primer

<400>    33
gcctttttgc aacttcggcg gcgccc                                             26

<210>    34
<211>    34
<212>    DNA
<213>    Artificial Sequence

<220>
<223>    primer

<400>    34
cgactctaga ggatccgtcg agcagggttt cctg                                    34

<210>    35
<211>    34
<212>    DNA
<213>    Artificial Sequence

<220>
<223>    primer

<400>    35
ctctacgccg ggatctgccg atgggttttt ccag                                    34

<210>    36
<211>    35
<212>    DNA
<213>    Artificial Sequence

<220>
<223>    primer

<400>    36
gcctttttgc ggatctgaaa agtcgagcga gatcg                                   35

<210>    37
<211>    33
<212>    DNA
<213>    Artificial Sequence

<220>
<223>    primer


<400>    37
cggtacccgg ggatcccaga cagtccctca tcg                                    33


<210>    38
<211>    31
<212>    DNA
<213>    Artificial Sequence

<220>
<223>    primer


<400>    38
agctgatcca gatctatgac gctgccattc g                                      31


<210>    39
<211>    32
<212>    DNA
<213>    Artificial Sequence

<220>
<223>    primer


<400>    39
aggcaaccta gatctggcat cccaaatatc cg                                     32


<210>    40
<211>    32
<212>    DNA
<213>    Artificial Sequence

<220>
<223>    primer


<400>    40
agctgatcca gatctatgaa ccgtaacggc ag                                     32


<210>    41
<211>    32
<212>    DNA
<213>    Artificial Sequence

<220>
<223>    primer


<400>    41
aggcaaccta gatctcgcat cccaaatatc cg                                     32


<210>    42
<211>    32

<212>    DNA
<213>    Artificial Sequence

<220>
<223>    primer


<400>    42
agctgatcca gatctatgac ccgtactgct cc                                    32


<210>    43
<211>    32
<212>    DNA
<213>    Artificial Sequence

<220>
<223>    primer


<400>    43
aggcaaccta gatctggcat cccaaatttc cg                                    32


<210>    44
<211>    301
<212>    PRT
<213>    Silicibacter pomeroyi

<400>    44
Met Asn Arg Asn Gly Ser Thr Lys Asp Pro Arg Arg Met Thr Gly Ala
1               5                   10                  15

Ala Thr Gly Glu Ile Ser Arg Tyr Phe Asn Asp Lys Ala Pro Lys Asp
            20                  25                  30

Ile Arg Arg Ala Ile Glu Lys Ala Asp Lys Asp Asp Ile Leu Ser Thr
        35                  40                  45

Thr Tyr Pro Tyr Asp Ala Glu Met Thr Ala Lys Asp Tyr Arg Ala Gln
    50                  55                  60

Met Glu Ala Leu Gln Ile Glu Leu Val Lys Leu Gln Ala Trp Ile Lys
65                  70                  75                  80

Gln Ser Gly Ala Arg Val Ala Leu Leu Phe Glu Gly Arg Asp Ala Ala
                85                  90                  95

Gly Lys Gly Gly Thr Ile Lys Arg Phe Arg Glu Asn Leu Asn Pro Arg
            100                 105                 110

Gly Ala Arg Val Val Ala Leu Ser Lys Pro Thr Glu Ala Glu Arg Ser
        115                 120                 125

Gln Trp Tyr Phe Gln Arg Tyr Ile Gln His Leu Pro Ser Ala Gly Glu
    130                 135                 140

Leu Val Phe Tyr Asp Arg Ser Trp Tyr Asn Arg Gly Val Val Glu His
145                 150                 155                 160

Val Phe Gly Trp Cys Asp Glu Glu Gln Arg Glu Arg Phe Phe Arg Gln
                165                 170                 175

Val Met Pro Phe Glu His Asp Leu Val Asp Asp Gly Ile His Leu Phe
            180                 185                 190

Lys Phe Trp Leu Asn Val Gly Arg Ala Glu Gln Leu Arg Arg Phe His
        195                 200                 205

Asp Arg Glu Arg Asp Pro Leu Lys Gln Trp Lys Leu Ser Pro Val Asp
    210                 215                 220

Ile Ala Gly Leu Asp Lys Trp Glu Ala Tyr Thr Thr Ala Ile Ser Gln
225                 230                 235                 240

Thr Leu Thr Arg Ser His Ser Asp Arg Ala Pro Trp Thr Val Ile Arg
                245                 250                 255

Ser Asp Asp Lys Lys Arg Ala Arg Leu Ala Ala Ile Arg Thr Val Leu
            260                 265                 270

Ser Gly Ile Asp Tyr Asp Asn Lys Asp Arg Ala Ala Val Gly Gln Pro
        275                 280                 285

Asp Ala Ala Ile Cys Gly Gly Pro Asp Ile Trp Asp Ala
    290                 295                 300

<210>   45
<211>   903
<212>   DNA
<213>   Silicibacter pomeroyi

<400>   45
atgaaccgta acggcagcac taaggaccct cgtcgtatga cgggtgcagc aactggtgag      60

atcagccgtt acttcaacga caaagccccg aaagacatcc gccgtgcaat cgagaaggca     120

gacaaagacg acatcctgtc caccacctac ccgtacgacg cagagatgac cgctaaagac     180

taccgtgcgc aaatggaagc tctgcagatc gaactggtca aactgcaggc gtggatcaag     240

cagagcggcg cacgtgtagc actgctgttc gaaggtcgtg acgctgctgg taaaggtggt     300

actatcaaac gtttccgtga gaacctgaac ccgcgtggcg ctcgtgtcgt ggctctgtct     360

aaaccaaccg aagctgaacg tagccagtgg tacttccagc gttacatcca gcacctgcca     420

tccgctggtg aactggtatt ctacgaccgc tcttggtata accgcggcgt ggtggaacac     480

gtgtttggtt ggtgcgacga agaacagcgt gaacgtttct tccgccaggt tatgccgttc     540

gaacacgacc tggttgacga cggtatccac ctgttcaaat tttggctgaa tgtaggccgc     600

gcggaacagc tgcgtcgctt tcatgaccgc gaacgcgatc cgctgaaaca gtggaaactg     660

tccccggttg atatcgcggg tctggataaa tgggaagcct ataccacggc gatctcccaa     720

accctgaccc gttcccattc tgatcgtgcc ccgtggactg ttattcgttc tgatgataag     780

aaacgtgcgc gcctggccgc gattcgcacc gttctgtctg gcattgatta tgataacaaa     840

gatcgcgcgg cggttggcca gccggatgcc gctatttgtg gcggcccgga tatttgggat     900

gcg                                                                 903

<210>   46
<211>   289
<212>   PRT
<213>   Silicibacter lacuscaerulensi

<400>   46
Met Thr Arg Thr Ala Pro Gly Ala Ile Asn Asp Tyr Phe Arg Asn His
1               5                   10                  15

Ala Pro Lys Asp Val Arg Gln Ala Ile Glu Asn Ala Gly Lys Asp Asp
            20                  25                  30

Ile Leu Asn Pro Ser Tyr Pro Tyr Ser Glu Arg Met Lys Gly Lys Val
        35                  40                  45

Tyr Asp Arg His Met Asp Ala Leu Gln Ile Glu Leu Val Lys Met Gln
    50                  55                  60

Ser Trp Val Lys Glu Thr Gly Gln Arg Ile Ala Ile Ile Phe Glu Gly
65                  70                  75                  80

Arg Asp Ala Ala Gly Lys Gly Gly Thr Ile Lys Arg Phe Arg Glu Asn
                85                  90                  95

Leu Asn Pro Arg Gly Ala Arg Asn Val Ala Leu Ala Lys Pro Thr Glu
            100                 105                 110

Ala Glu Arg Ser Gln Trp Tyr Phe Gln Arg Tyr Val Gln His Leu Pro
        115                 120                 125

Ser Ala Gly Glu Ile Val Phe Phe Asp Arg Ser Trp Tyr Asn Arg Gly
    130                 135                 140

Val Val Glu Asn Val Phe Gly Phe Cys Thr Pro Glu Glu Arg Glu Arg
145                 150                 155                 160

Phe Phe Arg Gln Val Leu Pro Leu Glu His Gly Phe Val Asn Asp Gly
                165                 170                 175

Ile Arg Leu Phe Lys Phe Trp Leu Asn Val Gly Arg Ala Glu Gln Leu
            180                 185                 190

Asn Arg Phe Leu Ala Arg Glu Asn Asp Pro Leu Lys Gln Trp Lys Leu
        195                 200                 205

Ser Pro Ile Asp Ile Ala Gly Leu Gly Lys Trp Asp Glu Tyr Thr Gln
    210                 215                 220

Ser Ile Ser Glu Thr Leu Thr Arg Ser His Ser Asp Ile Cys Pro Trp
225                 230                 235                 240

Thr Ile Val Arg Ser Asp Asp Lys Lys Arg Ala Arg Ile Ala Thr Ile
                245                 250                 255

Gln Thr Val Leu Ala Ala Leu Asp Tyr Asp Glu Lys Asp Pro Asp Val
            260                 265                 270

Val Gly Thr Pro Asp Pro Asn Ile Ala Gly Gly Pro Glu Ile Trp Asp
        275                 280                 285

Ala

<210> 47
<211> 867
<212> DNA
<213> Silicibacter lacuscaerulensi

<400> 47
atgacccgta ctgctcctgg tgcaatcaac gattacttcc gtaaccacgc gcctaaagac 60

gtgcgtcagg caatcgagaa cgcaggtaaa gacgacatcc tgaacccaag ctacccatac 120

tccgaacgca tgaaaggcaa agtctacgac cgccacatgg acgctctgca aatcgaactg 180

gtcaagatgc agagctgggt gaaagaaacg ggccagcgta tcgcaatcat cttcgaaggt 240

cgcgacgcag caggtaaagg tggtaccatc aagcgcttcc gtgagaacct gaacccgcgt 300

ggtgctcgta atgtggctct ggctaaaccg actgaagctg aacgttccca atggtacttc 360

cagcgttacg tgcagcacct gccgtctgcg ggtgaaattg tattcttcga ccgtagctgg 420

tataaccgtg gtgtggttga gaacgtattc ggcttctgca ccccggaaga acgtgaacgt 480

ttcttccgtc aggttctgcc gctggaacat ggttttgtta acgacggcat ccgcctgttt 540

aaattttggc tgaacgttgg ccgcgccgaa cagctgaacc gttttctggc ccgtgagaac 600

gacccgctga aacagtggaa actgtccccg attgacatcg cgggtctggg caaatgggat 660

gaatatacgc agtctatctc cgaaaccctg acccgttctc attctgacat ctgtccgtgg 720

accatcgtac gctctgatga taagaaacgc gcgcgcattg cgaccattca gactgttctg 780

gcggcgctgg attatgatga gaaagatccg gatgttgttg gcactccgga tccgaatatt 840

gccggcggcc cggaaatttg ggatgcc 867

<210> 48
<211> 289
<212> PRT
<213> Rhodobacterales bacterium

<400> 48
Met Thr Leu Pro Phe Asp Gly Ala Ile Ser Arg Tyr Tyr Glu Thr Gly
1               5                   10                  15

Ala Pro Glu Glu Ile Arg Lys Ala Ile Gln Thr Ala Asp Lys Asp Glu
            20                  25                  30

Ile Ile Thr Pro Ser Tyr Pro His Arg Glu Arg Met Ala Arg Lys Thr
        35                  40                  45

Tyr Glu Ala Glu Leu Glu Ala Leu Gln Ile Glu Leu Val Lys Met Gln
    50                  55                  60

Ala Trp Val Lys Ala Ser Gly Ala Arg Ile Ala Ile Val Leu Glu Gly
65                  70                  75                  80

Arg Asp Ala Ala Gly Lys Gly Gly Thr Ile Lys Arg Phe Arg Glu Asn

85                  90                  95

Leu Asn Pro Arg Gly Ala Arg Val Val Ala Leu Ser Lys Pro Ser Glu
            100                 105                 110

Glu Glu Gln Ser Gln Trp Tyr Phe Gln Arg Tyr Ile Gln His Leu Pro
        115                 120                 125

Ser Gly Gly Glu Ile Val Phe Phe Asp Arg Ser Trp Tyr Asn Arg Gly
    130                 135                 140

Val Val Glu Lys Val Phe Gly Phe Cys Thr Asp Asp Gln Arg Glu Arg
145                 150                 155                 160

Phe Phe His Gln Val Lys Gly Phe Glu Gln Ala Leu Val Asp Asp Gly
                165                 170                 175

Val Lys Leu Phe Lys Phe Trp Leu Asn Val Gly Arg Ala Glu Gln Leu
            180                 185                 190

Arg Arg Phe Leu Lys Arg Glu Ser Asp Pro Leu Lys Gln Trp Lys Leu
        195                 200                 205

Ser Pro Ile Asp Val Lys Gly Leu Glu Lys Trp Asp Glu Tyr Thr Ala
    210                 215                 220

Ala Ile Ser Glu Thr Leu Gln Arg Ser His Cys Pro Glu Ala Pro Trp
225                 230                 235                 240

Thr Ile Val Arg Ser Asp Gly Lys Arg Arg Ala Arg Leu Ala Ala Ile
                245                 250                 255

Arg Ala Val Leu Ser Gly Ile Asp Tyr Asp Asn Lys Asp Ala Lys Ala
            260                 265                 270

Ile Gly Ala Leu Asp Thr Glu Ile Cys Gly Gly Pro Asp Ile Trp Asp
        275                 280                 285

Ala

<210>    49
<211>    867
<212>    DNA
<213>    Rhodobacterales bacterium

<400>    49
atgacgctgc cattcgatgg tgctatcagc cgttactacg aaacgggtgc acctgaagaa      60

atccgtaagg ctatccagac tgctgacaaa gacgagatca tcaccccatc ctacccgcac     120

cgtgaacgta tggcacgtaa gacctacgaa gctgaactgg aagcgctgca gatcgaactg     180

gtcaagatgc aggcatgggt caaggctagc ggtgctcgta tcgctatcgt actggaaggt     240

cgtgacgctg caggtaaagg tggtactatc aaacgcttcc gtgagaacct gaacccgcgt     300

ggtgcacgtg tagttgcact gtctaaaccg tctgaagagg agcagtccca gtggtacttc     360

cagcgctaca tccagcacct gccgtctggt ggcgaaatcg tattcttcga ccgttcctgg     420

tataaccgcg gtgtggtgga gaaagtgttc ggtttctgca ccgacgacca gcgtgaacgc     480

tttttccacc aggtgaaagg cttcgaacag gcactggttg acgacggcgt taaactgttt 540

aaattttggc tgaatgttgg ccgcgcggaa cagctgcgcc gttttctgaa acgcgaatcc 600

gacccgctga aacaatggaa actgtccccg attgacgtta aaggcctgga gaaatgggat 660

gaatataccg ccgcgatcag cgaaaccctg caacgcagcc attgcccgga agcgccgtgg 720

actatcgttc gttctgatgg caaacgtcgc gcgcgtctgg cggccattcg tgccgttctg 780

tctggcattg attatgataa caaagatgcg aaagcgattg gcgcgctgga taccgaaatt 840

tgtggcggcc cggatatttg ggatgcc 867

<210> 50
<211> 2533
<212> DNA
<213> E. coli

<400> 50
atgtcgcaag ataacaactt tagccagggg ccagtcccgc agtcggcgcg gaaaggggta 60

ttggcattga cgttcgtcat gctgggatta accttctttt ccgccagtat gtggaccggc 120

ggcactctcg gaaccggtct tagctatcat gatttcttcc tcgcagttct catcggtaat 180

cttctcctcg gtatttacac ttcatttctc ggttacattg gcgcaaaaac cggcctgacc 240

actcatcttc ttgctcgctt ctcgtttggt gttaaaggct catggctgcc ttcactgcta 300

ctgggcggaa ctcaggttgg ctggtttggc gtcggtgtgg cgatgtttgc cattccggtg 360

ggtaaggcaa ccgggctgga tattaatttg ctgattgccg tttccggttt actgatgacc 420

gtcaccgtct tttttggcat ttcggcgctg acggttcttt cggtgattgc ggttccggct 480

atcgcctgcc tgggcggtta ttccgtgtgg ctggctgtta acggcatggg cggcctggac 540

gcattaaaag cggtcgttcc cgcacaaccg ttagatttca atgtcgcgct ggcgctggtt 600

gtggggtcat ttatcagtgc gggtacgctc accgctgact ttgtccggtt tggtcgcaat 660

gccaaactgg cggtgctggt ggcgatggtg gcctttttcc tcggcaactc gttgatgttt 720

attttcggtg cagcgggcgc tgcggcactg ggcatggcgg atatctctga tgtgatgatt 780

gctcagggcc tgctgctgcc tgcgattgtg gtgctggggc tgaatatctg gaccaccaac 840

gataacgcac tctatgcgtc gggtttaggt ttcgccaaca ttaccgggat gtcgagcaaa 900

accctttcgg taatcaacgg tattatcggt acggtctgcg cattatggct gtataacaat 960

tttgtcggct ggttgacctt cctttcggca gctattcctc cagtgggtgg cgtgatcatc 1020

gccgactatc tgatgaaccg tcgccgctat gagcactttg cgaccacgcg tatgatgagt 1080

gtcaattggg tggcgattct ggcggtcgcc ttggggattg ctgcaggcca ctggttaccg 1140

ggaattgttc cggtcaacgc ggtattaggt ggcgcgctga gctatctgat ccttaacccg 1200

attttgaatc gtaaaacgac agcagcaatg acgcatgtgg aggctaacag tgtcgaataa 1260

cgctttacaa acaattatta acgcccggtt accaggcgaa gaggggctgt ggcagattca 1320

tctgcaggac ggaaaaatca gcgccattga tgcgcaatcc ggcgtgatgc ccataactga 1380

aaacagcctg gatgccgaac aaggtttagt tataccgccg tttgtggagc cacatattca 1440

cctggacacc acgcaaaccg ccggacaacc gaactggaat cagtccggca cgctgtttga 1500

aggcattgaa cgctgggccg agcgcaaagc gttattaacc catgacgatg tgaaacaacg 1560

cgcatggcaa acgctgaaat ggcagattgc caacggcatt cagcatgtgc gtacccatgt 1620

cgatgtttcg gatgcaacgc taactgcgct gaaagcaatg ctggaagtga agcaggaagt 1680

cgcgccgtgg attgatctgc aaatcgtcgc cttccctcag gaagggattt tgtcgtatcc 1740

caacggtgaa gcgttgctgg aagaggcgtt acgcttaggg gcagatgtag tgggggcgat 1800

tccgcatttt gaatttaccc gtgaatacgg cgtggagtcg ctgcataaaa ccttcgccct 1860

ggcgcaaaaa tacgaccgtc tcatcgacgt tcactgtgat gagatcgatg acgagcagtc 1920

gcgctttgtc gaaaccgttg ctgccctggc gcaccatgaa ggcatgggcg cgcgagtcac 1980

cgccagccac accacggcaa tgcactccta taacggggcg tatacctcac gcctgttccg 2040

cttgctgaaa atgtccggta ttaactttgt cgccaacccg ctggtcaata ttcatctgca 2100

aggacgtttc gatacgtatc caaaacgtcg cggcatcacg cgcgttaaag agatgctgga 2160

gtccggcatt aacgtctgct ttggtcacga tgatgtcttc gatccgtggt atccgctggg 2220

aacggcgaat atgctgcaag tgctgcatat ggggctgcat gtttgccagt tgatgggcta 2280

cgggcagatt aacgatggcc tgaatttaat cacccaccac agcgcaagga cgttgaattt 2340

gcaggattac ggcattgccg ccggaaacag cgccaacctg attatcctgc cggctgaaaa 2400

tgggtttgat gcgctgcgcc gtcaggttcc ggtacgttat tcggtacgtg gcggcaaggt 2460

gattgccagc acacaaccgg cacaaaccac cgtatatctg gagcagccag aagccatcga 2520

ttacaaacgt tga 2533